# EUROPEAN PATENT SPECIFICATION

(11) **EP 4 233 735 B1**
(45) Date of publication and mention of the grant of the patent: **03.12.2025**
(21) Application number: 23175244.5
(22) Date of filing: 04.05.2020
(51) Int. Cl.: A61B 17/00

(54) **CROSSABLE INTERSEPTAL OCCLUDER DEVICE**
DURCHQUERBARE INTERSEPTALE VERSCHLUSSVORRICHTUNG
DISPOSITIF D'OCCLUSION INTERSEPTALE POUVANT ÊTRE TRAVERSÉ

(30) Priority: 03.05.2019 IT 201900006534
(43) Date of publication of application: 30.08.2023
(62) Divisional of application: 20742445.8
(73) Proprietor: RECROSS CARDIO, INC., Kent County, DE 19901 (US)
(72) Inventor: ROLANDO, Giovanni, Dover Kent County, DE 19901 (US); ANTONUCCI, Marta, Dover Kent County, DE 19901 (US); ROGGERO, Simona, Dover Kent County, DE 19901 (US)
(74) Representative: Crippa, Paolo Ernesto

(56) References cited:
- US-A1- 2007 073 337
- US-A1- 2014 012 368
- US-A1- 2016 022 423
- US-A1- 2016 296 684

## Description

### . Field of the invention

**.** In the most general aspect thereof, the present invention is directed to an occluder device. In particular, the present invention relates to an interseptal occluder device. Even more particularly, the present invention relates to an interseptal occluder device of the crossable type, or crossable interseptal occluder device.

**.** In particular, the present invention is directed to a device for closing a defect of a partition or an opening obtained in a partition, not necessarily of the heart.

**.** Even more particularly, the present invention is directed to a device for closing a defect present in a partition, for example a defect in an atrial partition, so that the same defect, even if occluded, can then be used for a medical device to pass through said defect.

**.** Moreover, the device of the present invention is intended to occlude defects (typically congenital defects, but not only) or interatrial partition holes/openings created following percutaneous interventions with trans-septal puncture techniques (for example for the mitral valve repair or the occlusion of the Left Atrial Appendage) .

### . Background art

**.** A partition is for example a thin wall dividing a cavity into two smaller cavities or chambers or compartments. The term "partition" is intended to define both a heart wall which divides two atria, as well as a wall which divides the right or left atrium and ventricle.

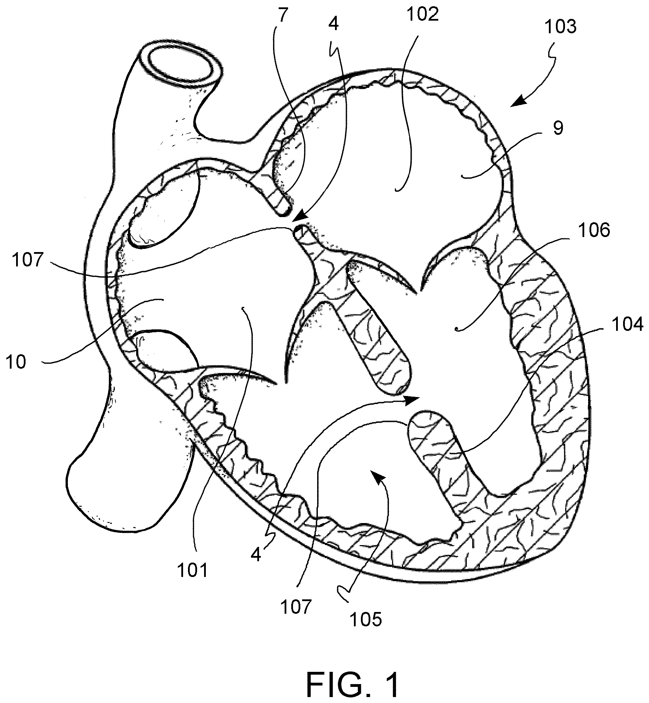

**.** With reference to Figure 1, an atrial partition 100 is a tissue wall which separates the right atrium 101 from the left atrium 102 of the heart 103.

**.** A ventricular partition 104 is a tissue wall which separates the right 105 and left 106 ventricles of the heart 103.

**.** A defect 107 of the partition 100, 104 may include a perforation or a hole in the partition. A defect 107 of the partition 100, 104 can occur congenitally or by piercing the partition with a medical device to access a position within the heart.

**.** The femoral vein is an access point for many laboratory catheterization procedures, with a smaller percentage of procedures using the access to arteries.

**.** The atrial partition 100 is a percutaneous access point, for example for atrial fibrillation therapy, closure of the left atrial appendage, percutaneous repair of the mitral valve, and percutaneous replacement of the mitral valve. In these and other procedures, the devices need to cross the atrial partition 100 and, in doing so, can leave an orifice in the atrial partition which cannot close or heal on its own.

**.** Therefore, these defects are often closed using devices, such as clips or occluders. However, these devices do not allow re-crossing through the partition.

**.** Therefore, there is a need for improved occlusion devices for closing a defect or an opening of the partition, and for re-crossing it in (possible) subsequent procedures.

**.** Crossable occluding devices are known from the prior art. These have an anchoring structure and a diaphragm or valve connected thereto.

. For example, documents WO2017136287A1**,** US2014074155 show crossable occluder solutions.

**.** Document US20070073337 shows devices and methods for occluding the defects of internal tissues, such us the defects of the partition, with clip-based devices. A device having a clip structure is shown, which includes a tubular body having at least a first and a second deflectable element coupled thereto. The first and second elements are coupled at the opposite ends of the tubular body and configured to switch from a non-deployed configuration to a deployed configuration. In the deployed configuration, each element extends outwardly away from the tubular body in a position configured to rest on a tissue surface. The first and second elements of the clip are preferably configured to support a tissue wall therebetween and close any opening in the tissue wall. This solution does not allow to re-cross the device once implanted.

. Document US20140012368 shows devices and methods for improving the implantation, retractability, or repositioning of a device for positioning a valve in a partition. The embodiments of the devices include pivotable sections which provide the ability to maintain the engagement of the device with a release system during implantation, in which the release system approaches an opening of the partition. The embodiments of the devices include configurations which allow for better recovery in a delivery system if a malfunction or problem with the patient's physiology is detected. This device is used to implant a flow control valve. These solutions are devices for treating heart failure. In particular, it is intended to create interatrial pressure outlets, shunts, and the like, which reduce the high pressure on one side of the heart, thus mitigating the resulting symptoms. Therefore, this solution aims to create an opening in the partition. This flow control element is a tissue valve such as a tricuspid valve, a bicuspid valve or a single-leaflet valve made of pericardial tissue from cattle, pigs, sheep or other animals, and therefore it has cusp-like foils made of preferably natural tissue and cusp-shaped.

**.** Document US20160296684 discloses an endoluminal device with an anchor having first and second anchoring portions. The device includes a central body and fixed vanes. Document US 2016/022423 A1 describes an insert for an interatrial device featuring a plate of biocompatible plastic with a plurality of orifices and flaps sewn over the orifices with bioabsorbable sutures. These orifices must permit blood flow when implanted, with any orifices in the shape of an aperture between two bridges.

**.** These solutions, although being advantageous in some aspects, are very complex to implement, and especially do not allow to substantially completely occlude the partition defect while choosing freely the crossing point or re-crossing point in different positions of the diaphragm, thus facilitating the approach of the surgical instrument to the surgery area which can be located very differently from patient to patient and from disease to disease.

**.** Therefore, the need for an occluding device which is simple to manufacture, easy to apply, and flexible to use after implantation for crossing the defect or the now-occluded opening of the partition in case of subsequent surgery is still strongly felt.

### . Solution

**.** Therefore, it is the object of the present invention to provide a crossable interseptal occluder device, having structural and functional features such as to meet the aforementioned needs and overcome the drawbacks mentioned above with reference to the devices of the prior art.

**.** These and other objects are achieved by a device according to claim **1.**

**.** Some advantageous embodiments are the subject of the dependent claims.

### . Drawings

. Further features and advantages of the invention will become apparent from the description provided below of preferred exemplary embodiment thereof, given by way of non-limiting example, with reference to the accompanying drawings, in which:
- Figure 1 is a diagrammatic sectional view of a heart in which defects or openings are present in the atrial and ventricular partitions;

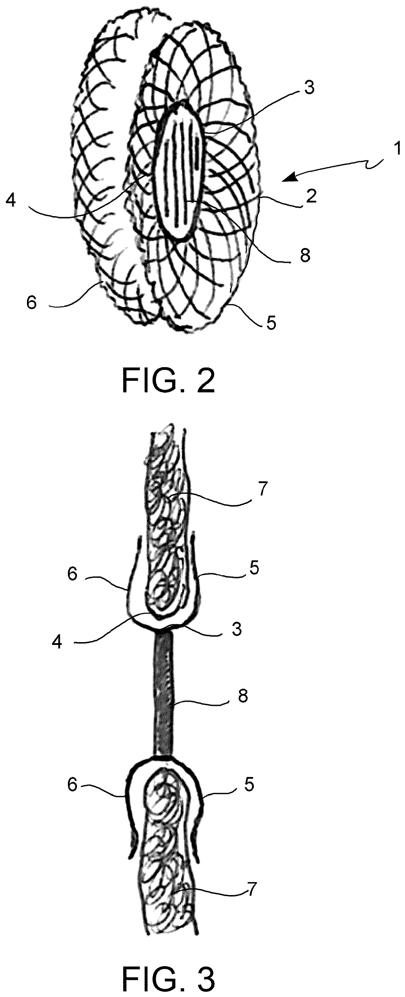

- Figure 2 shows an axonometric view of a crossable interseptal occluding device according to the present invention;
- Figure 3 is a cross-sectional view of a crossable interseptal occluding device applied to close a defect or opening of a partition;
- Figure 4A is a diagrammatic front view of only the part of the diaphragm of a crossable interseptal occluding device, made according to an embodiment with parallel fringes;
- Figure 4B is a diagrammatic front view of only the part of the diaphragm of a crossable interseptal occluding device, made according to an embodiment with radial fringes;
- Figure 5 is a diagrammatic front view of only the part of the diaphragm of a crossable interseptal occluding device, made according to an embodiment where two diaphragms at least partially overlap with each other and the fringes thereof, arranged parallel in the same diaphragm, are oriented orthogonal to the facing diaphragm;
- Figure 6 is a perspective view of only the part of the diaphragm of a crossable interseptal occluding device crossed by a medical device, here depicted as a tubular body only, by moving away and extending the fringes of said diaphragm;
- Figures 7A to 7E depict only the part of the diaphragm of a crossable interseptal occluding device, in a front view and in a local cross section of the fringes alone in order to show the shape of the sections of the fringes according to four different embodiments;
- Figures 8A and 8B show only the part of the diaphragm of a crossable interseptal occluding device, in a front view and in a local cross section of the fringes alone according to an embodiment with tapered fringes;
- Figure 9 depicts only the local cross sections of two diaphragms with tapered fringes, in a facing phase, in which one diaphragm is rotated and offset from the second one, so as to arrange the tapered fringes thereof between the tapered fringes of the facing diaphragm and mutually partially interpenetrated;

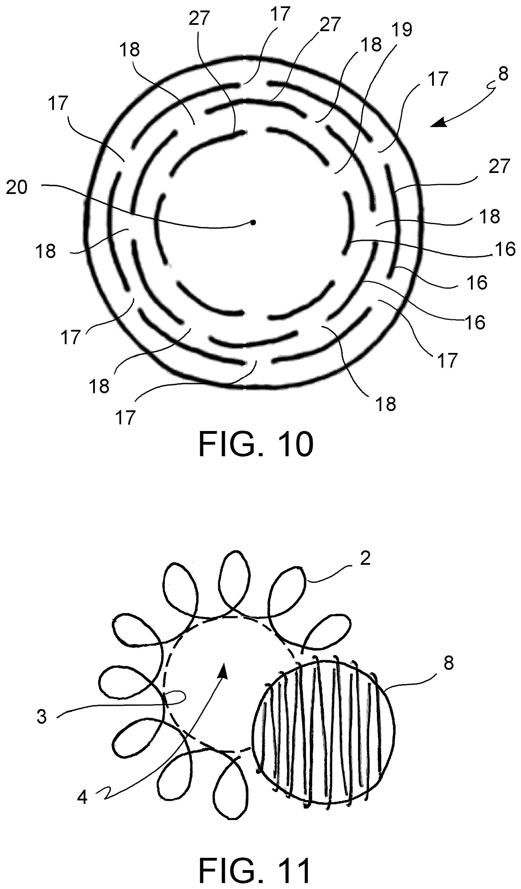

- Figure 10 is a diagrammatic front view of only the part of the diaphragm of a crossable interseptal occluding device, made according to an embodiment with radial fringes and annular path interrupted by small bridges;
- Figure 11 is a diagrammatic front view of an operation for connecting a diaphragm to a support structure to obtain a crossable interseptal occluding device;
- Figure 12 depicts a diaphragm of a crossable interseptal occluding device made with a single wire folded and forming wire stretches which are mutually substantially parallel;
- Figure 13 is a diagrammatic front view of only the part of the diaphragm of a crossable interseptal occluding device, made according to an embodiment where two diaphragms at least partially overlap with each other and the fringes thereof, shaped as wires, are arranged parallel in the same diaphragm, but are oriented orthogonal to the facing diaphragm;

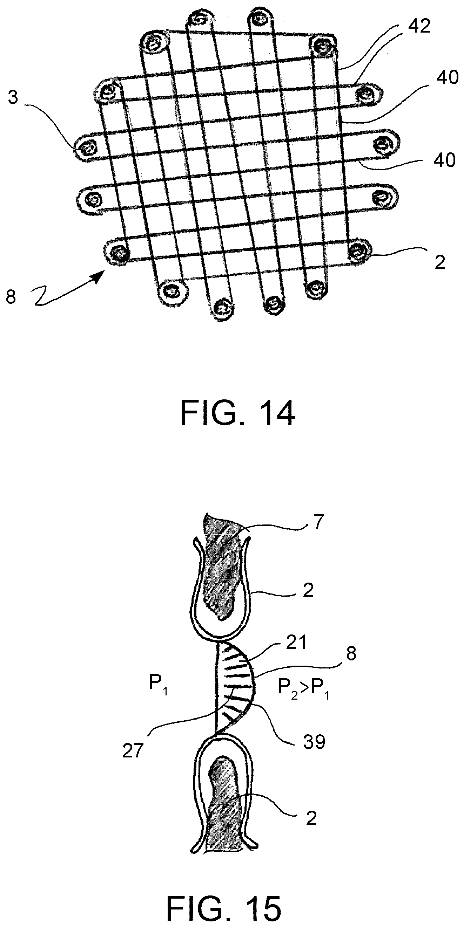

- Figure 14 depicts a diaphragm of a crossable interseptal occluding device made with wires folded and forming first wire stretches which are mutually substantially parallel and second stretches which are substantially parallel but transverse to the first stretches;
- Figure 15 is a cross-sectional view of a crossable interseptal occluding device applied to close a defect or opening of a partition, in which the diaphragm is dome-shaped with concavity facing the cavity or compartment where a higher pressure is expected;

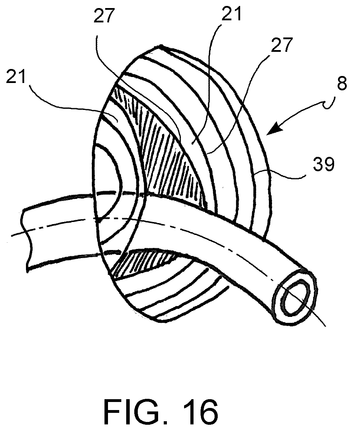

- Figure 16 is an axonometric view of a diaphragm of a crossable interseptal occluding device, in which the diaphragm is dome-shaped with concavity facing the cavity or compartment where a higher pressure is expected and is crossed by a medical device, here depicted in a tubular shape only, which moves and extends the fringes;

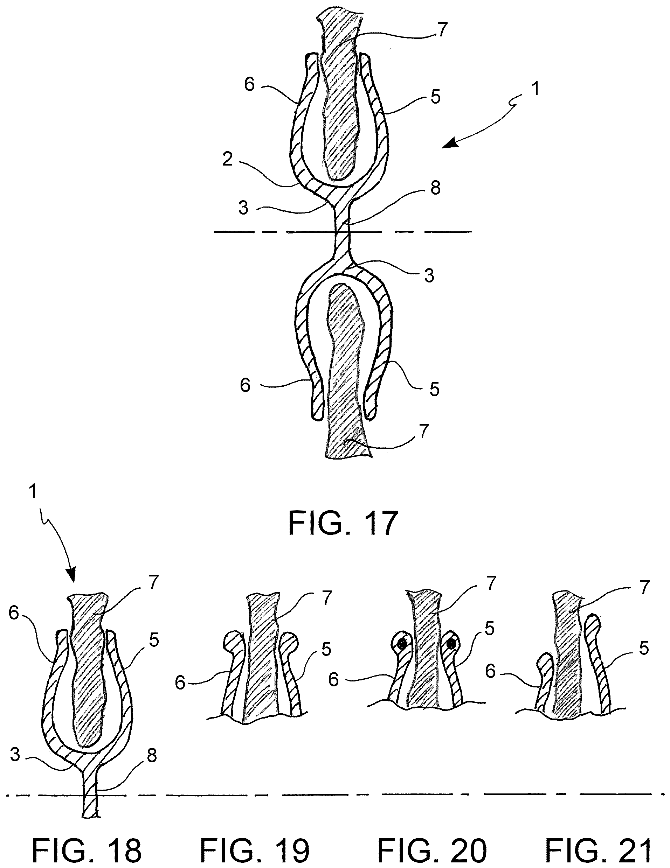

- Figure 17 shows a cross-sectional views of a crossable interseptal occluding device applied to close a defect or opening of a partition, in which the diaphragm is in one piece with the support structure anchored to the partition;
- Figures 18 to 21 show cross-sectional views of different embodiments of the anchoring portions of a support structure applied to a defect or opening of a partition and made in a single piece with the diaphragm;
- Figures 22 to 24 show a front view of different embodiments of anchoring portions of support structures of crossable interseptal occluding devices, in particular disc-shaped, three-lobed and angularly offset, six-lobed and angularly offset;
- Figure 25 is a front view of a crossable interseptal occluding device in which at least one of the anchoring portions of a support structure has circumferentially distributed openings;
- Figure 26 shows a diagrammatic front view of only the part of the diaphragm of a crossable interseptal occluding device, made according to an embodiment with fringes and wavy fringe edges or fringes with wavy fringe body, which is suitable for a greater elastic extension in case of re-crossing by a medical device;
- Figure 27 is a diagrammatic axonometric view of only the diaphragm of the crossable interseptal occluding device, in which the occluding bridges are according to a further embodiment;
- Figure 28 shows an axonometric sectional view according to line XXVII-XXVII in figure 27 in which the shapes of the cross sections of the occluding bridges are highlighted, showing a meshing or geometric coupling between the different elongated bodies of the occluding bridges, thus creating slits or cuts for separating the bodies of the bridges with a winding shape;
- Figure 29 is a front view of a diaphragm of a crossable interseptal occluding device, according to a further embodiment, where the occluding bridges have independent leaves in the central body portion thereof and inclined with respect to the plane of the diaphragm or lumen, so as to create winding path separation slits, for example by partially overlapping a bridge with the adjacent one;
- Figure 30 shows a section ac-cording to line XXX-XXX in Figure 29 of the diaphragm in Figure 29;

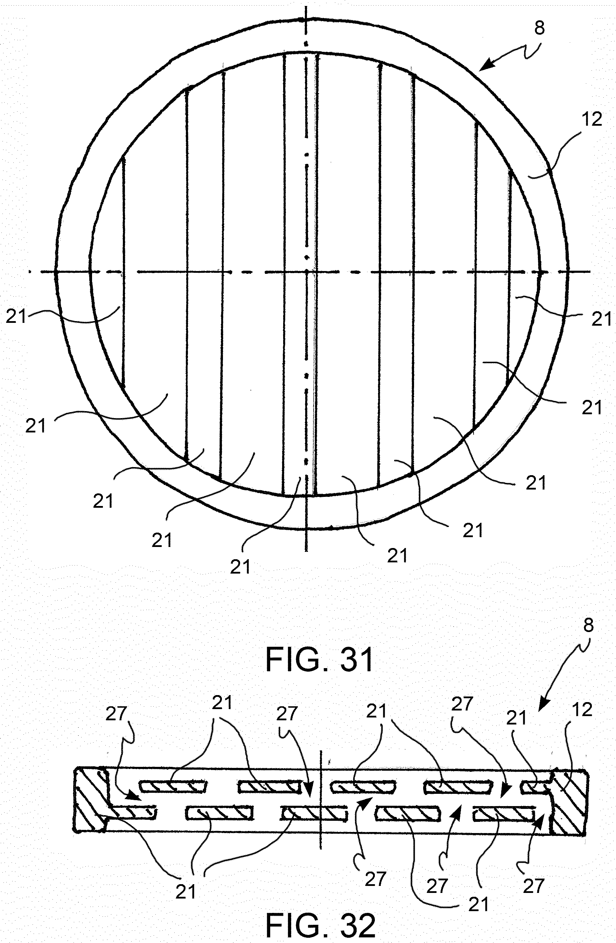

- Figure 31 is a front view of a diaphragm of a crossable interseptal occluding device, ac-cording to a further embodiment, where the occluding bridges have independent leaves in the central body portion thereof, which are arranged so as to be offset on two planes, alternatively on a first plane and on a second plane which is offset from and parallel to the first one, so as to create winding path separation slits, for example by partially overlapping a bridge with the adjacent one;
- Figure 32 shows a section ac-cording to line XXXII-XXXII in Figure 31 of the diaphragm in Figure 31;

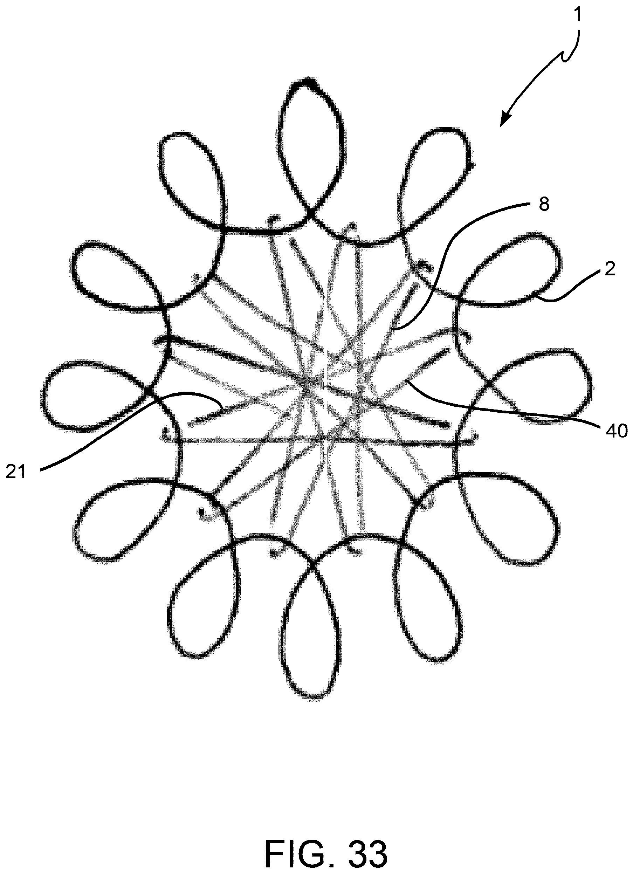

- Figure 33 is a diagrammatic view of a crossable interseptal occluding device in which the diaphragm is made from elements or a single thread-like element wrapped around the support structure, such as a tube, or an elastic thread, arranged so as to causally cross the area of the lumen and create a substantial occlusion in the majority of the lumen.

### . Description of some preferred embodiments

**.** In accordance with a general embodiment, there is provided a crossable interseptal occluder device 1 comprising a support structure 2 in which said support structure 2 comprises a central support structure portion 3 which delimits a lumen 4.

**.** Said support structure 2 comprises a first anchoring portion 5 and an opposite second anchoring portion 6.

**.** Said support structure 2 being configured to expand and contract between a compressed tubular configuration for the insertion through the patient's vasculature and an expanded or extended configuration in which the first and second anchoring portions 5, 6 extend radially outwards from said central support structure portion 3 to compress a partition 7 therebetween by arranging said support structure 2 astride said partition 7 through a defect or hole or opening present in the partition 7.

**.** Said crossable interseptal occluder device 1 further comprises at least one diaphragm 8.

**.** Said diaphragm 8 being supported by said support structure 2 and arranged to close the majority of said lumen 4, when said diaphragm 8 is in a relaxed configuration with the supporting structure 2 being extended.

**.** Said diaphragm 8 being configured to allow a medical instrument inserted into a first compartment 9 or 10 delimited by said septum 7 to pass through the diaphragm 8 and then through said lumen 4 entering a second compartment 10 or 9 delimited by said septum 7.

**.** Said diaphragm 8 comprises a diaphragm edge 11 placed close to said central support structure portion 3 of said support structure 2.

**.** Said diaphragm 8 comprises a plurality of elongated membrane occluding fringes or bridges 21 or occluding bridges 21.

**.** Each of said occluding bridges 21 comprises an elongated body 50 having opposite elongated body ends or ends 51, 52 and opposite longitudinal edges or sides 22.

**.** In a relaxed configuration of said diaphragm 8, each occluding bridge 21 of said plurality of occluding bridges 21 is arranged close to at least a further occluding bridge 21 of said plurality of occluding bridges 21.

**.** At least one portion of said longitudinal sides 22 of each occluding bridge 21 and at least one portion of said longitudinal sides 22 of said at least a further occluding bridge 21 delimit at least one slit or one cut 27 into said diaphragm 8.

**.** Both said opposite ends 51, 52 of each occluding bridge 21 are directly or indirectly connected to or supported by said support structure 2.

**.** The term "occlude" means the possibility of minimizing or completely avoiding the flow of blood passing through said diaphragm. This definition is to describe an occlusion created by a plurality of occluding elements which, arranged to cover the lumen, create barriers while remaining at least partially independent of each other to be movable when necessary, but which tend to return, when not urged, to the occlusion position.

**.** The term "relaxed position or configuration of the diaphragm" means a configuration both unimplanted and implanted in the patient in which the support structure is expanded or deployed or extended in which the diaphragm is not urged by external forces, but not necessarily without internal actions, such as a tension which tends to keep the occluding bridges in the occlusion position. For example, this position coincides with that of the support structure in an extended position, adapted to grasp the edges of the partition close to the edge of the lumen to be occluded. For example, but not necessarily, the relaxed position of the diaphragm coincides with an arrangement of the occluding bridges which is planar as a whole or on planes.

**.** In accordance with an embodiment, in a relaxed configuration of said diaphragm 8, each of said longitudinal sides 22 delimits, with the side of an adjacent occluding bridge 21, an elongated and narrow opening or elongated slit 27.

**.** In accordance with an embodiment, in a relaxed configuration of said diaphragm 8, each bridge of said plurality of occluding bridges 21 of each diaphragm 8 of said at least one diaphragm 8 lies in a single plane.

**.** In accordance with an embodiment, in a relaxed configuration of said diaphragm 8, said plurality of occluding bridges 21 is arranged with the bridges placed side by side.

**.** In accordance with an embodiment, in a relaxed configuration of said diaphragm 8, each occluding bridge 21 is arranged close to at least a further occluding bridge 21 facing at least one portion of a longitudinal side 22 thereof to at least one portion of a longitudinal side 822 of said further occluding bridge 21.

**.** In accordance with an embodiment, in a relaxed configuration of said diaphragm 8, said slit or cut 27 is one elongated slit 27; and/or wherein said slit or cut 27 is a long and narrow opening 11 delimited by occluding bridges 21.

**.** In accordance with an embodiment, in a relaxed configuration of said diaphragm 8, said each occluding bridge 21 is placed adjacent to said at least a further occluding bridge 21.

**.** In accordance with an embodiment, in a relaxed configuration of said diaphragm 8, each of said opposite longitudinal sides 22 lies so that the sum of the elongated bodies 50 of said occluding bridges 21 occludes the majority of said lumen 4.

**.** In accordance with an embodiment, in a relaxed configuration of said diaphragm 8, said each occluding bridge 21 is arranged parallel to said at least a further occluding bridge 21.

**.** In accordance with an embodiment, in a relaxed configuration of said diaphragm 8, said slit or cut 27 has a longitudinal extension equal to the entire longitudinal extension of said elongated body 50 of said each occluding bridge 21 which delimits said slit or cut 27.

**.** In accordance with an embodiment, in a relaxed configuration of said diaphragm 8, all slits or cuts 27 are arranged parallel to one another.

**.** In accordance with an embodiment, except for the opposite elongated body ends 51, 52, each elongated body 50 of each occluding bridge 21 is independent of other elongated bodies 50 of other occluding bridges 21.

**.** In accordance with an embodiment, in a relaxed configuration of said diaphragm 8, the occluding bridges 21 of said plurality of occluding bridges 21 are randomly distributed in order to occlude the majority of said lumen 4.

**.** In accordance with an embodiment, in a relaxed configuration of said diaphragm 8, the occluding bridges 21 of said plurality of occluding bridges 21 are arranged so as to avoid from intertwining or interlacing with one another.

**.** In accordance with an embodiment, in a relaxed configuration of said diaphragm 8, each of said bridges of said plurality of occluding bridges 21 lies in a plane arranged orthogonal to an axis of said lumen 4.

**.** In accordance with an embodiment, in a relaxed configuration of said diaphragm 8, all bridges of said plurality of occluding bridges 21 of each diaphragm 8 lie in the same plane.

**.** In accordance with an embodiment, both said opposite ends 51, 52 of each occluding bridge 21 are directly or indirectly connected to or supported by said central support structure portion 3 of said support structure 2.

**.** In accordance with an embodiment, said opposite longitudinal sides 22 of said plurality of occluding bridges 21 are parallel to one another.

. In accordance with an embodiment, said plurality of occluding bridges 21 comprises opposite longitudinal sides 22 and where said opposite longitudinal sides 22 of adjacent occluding bridges 21 are arranged side by side and in contact with one another.

. In accordance with an embodiment, said diaphragm 8 is made of an elastic material, for example silicone or medical elastomer or polyurethane or bioerodible or bioabsorbable material.

. In accordance with an embodiment, said diaphragm 8 is made of an elastic material capable of deformation at least between 10% and 20%.

**.** In accordance with the invention, said diaphragm 8 is made of an elastic material, for example silicone or medical elastomer or polyurethane or bioerodible or bioabsorbable material; and where said diaphragm 8 is made of an elastic material capable of deformation of at least 150%.

**.** In accordance with an embodiment, said diaphragm 8 is an elastic membrane.

**.** In accordance with an embodiment, said occluding bridges 21 of said diaphragm 8 are separated from one another in the extension thereof or central occluding bridge portion 29 but joined together in a single piece.

**.** In accordance with an embodiment, said diaphragm edge 11 and said occluding bridges 21 are in a single piece.

**.** In accordance with an embodiment, at least one of said occluding bridges 21 has a longitudinal extension and comprises an occluding bridge section 23 evaluated in a transverse direction to said longitudinal direction of circular shape.

**.** In accordance with an embodiment, at least one of said occluding bridges 21 has a longitudinal extension and comprises an occluding bridge section 23 evaluated in a transverse direction to said longitudinal direction of square or rectangular shape.

**.** In accordance with an embodiment, at least one of said occluding bridges 21 has a longitudinal extension and comprises an occluding bridge section 23 evaluated in a transverse direction to said longitudinal direction of tapered shape passing from the center of said section to a section end 24 thereof.

**.** In accordance with an embodiment, at least one of said occluding bridges 21 has a longitudinal extension and comprises an occluding bridge section 23 evaluated in a transverse direction to said longitudinal direction of tapered shape passing from a first section base side 25 to a section end 24 thereof.

**.** In accordance with an embodiment, at least one of said occluding bridges 21 has a longitudinal extension and comprises an occluding bridge section 23 evaluated in a transverse direction to said longitudinal direction of tapered shape passing from a first section base side 25 to smaller section side 26.

**.** In accordance with an embodiment, at least one of said occluding bridges 21 has a longitudinal extension and comprises an occluding bridge section 23 evaluated in a transverse direction to said longitudinal direction of oval or triangular or rhomboidal or trapezoidal shape.

**.** In accordance with an embodiment, at least one pair of said occluding bridges 21 has a longitudinal extension and comprises an occluding bridge section 23 evaluated in a transverse direction to said longitudinal direction of concave shape, in the first occluding bridge 21 of said pair, facing a convex shape, in the second occluding bridge 21 of said pair, and said concave and convex shapes are at least partially meshed with each other.

**.** In accordance with an embodiment, at least one pair of said occluding bridges 21 has a longitudinal extension and comprises an occluding bridge section 23 evaluated in a transverse direction to said longitudinal direction of concave shape, in the first occluding bridge 21 of said pair, facing a convex shape, in the second occluding bridge 21 of said pair, and said concave and convex shapes are at least partially meshed with each other and said shapes are arched shapes.

**.** In accordance with an embodiment, at least one pair of said occluding bridges 21 has a longitudinal extension and comprises an occluding bridge section 23 evaluated in a transverse direction to said longitudinal direction of concave shape, in the first occluding bridge 21 of said pair, facing a convex shape, in the second occluding bridge 21 of said pair, and said concave and convex shapes are at least partially meshed with each other and said shapes are triangular or trapezoidal shapes.

**.** In accordance with an embodiment, a pair of diaphragms 28 is included, each of said diaphragms 8 of said pair of diaphragms 28 comprising a plurality of occluding bridges 21.

**.** Said diaphragms 8 of said pair of diaphragms 28 are arranged facing each other and with the plurality of occluding bridges 21 of the first diaphragm 8 of said pair of diaphragms 28 being offset with respect to the plurality of diaphragm occluding bridges 21 of the second diaphragm 8 of said pair of diaphragms 28 by overlapping at least partially said plurality of occluding bridges 21 of the first diaphragm 8 to said plurality of elongated slits or cuts 27 of said second diaphragm 8.

**.** In accordance with an embodiment, said plurality of occluding bridges 21 of the first diaphragm 8 has tapered occluding bridge sections 23, for example triangular or rhomboidal in shape.

**.** The tapering of the occluding bridge sections 23 of said plurality of occluding bridges 21 of the first diaphragm 8 tapers towards said second diaphragm 8.

**.** In accordance with an embodiment, said plurality of occluding bridges 21 of the first diaphragm 8 interpenetrates at least partially between said occluding bridges 21 of said plurality of occluding bridges 21 of the second diaphragm 8.

**.** In accordance with an embodiment, said diaphragm 8 has a concave body or concavity facing one of said first or second compartment 9, 10.

**.** In accordance with an embodiment, said diaphragm 8 is dome-shaped.

**.** In accordance with an embodiment, said diaphragm 8 comprises a diaphragm stiffening structure 30.

**.** In accordance with an embodiment, said diaphragm 8 comprises a diaphragm stiffening structure 30, said diaphragm stiffening structure 30 comprises diaphragm ribs 31 adapted to create a support scaffold.

**.** In accordance with an embodiment, said diaphragm 8 comprises a diaphragm stiffening structure 30, said diaphragm stiffening structure 30 comprises diaphragm ribs 31 in a single piece with said diaphragm 8.

**.** In accordance with an embodiment, said diaphragm 8 comprises a diaphragm stiffening structure 30, said diaphragm stiffening structure 30 comprises at least one diametrical rib 32 extending along a diameter of said diaphragm 8.

**.** In accordance with an embodiment, said diaphragm 8 comprises a diaphragm stiffening structure 30, said diaphragm stiffening structure 30 comprises at least one circumferentially extending rib 33.

**.** In accordance with an embodiment, said diaphragm 8 comprises a diaphragm stiffening structure 30, said diaphragm stiffening structure 30 comprises at least one radially extending rib 34.

**.** In accordance with an embodiment, said diaphragm 8 comprises a diaphragm stiffening structure 30, said diaphragm stiffening structure 30 comprises at least one rib 35 extending along a circle chord.

**.** In accordance with an embodiment, said diaphragm 8 comprises a diaphragm stiffening structure 30, said diaphragm stiffening structure 30 comprises diaphragm ribs 31 adapted to create a support frame and said at least one rib is of elastic and/or extensible shape or material.

**.** In accordance with an embodiment, said diaphragm 8 comprises a first diaphragm portion 12 having an external continuous annular portion directly or indirectly connected to said support structure 2.

**.** Each occluding bridge end 51, 52 is connected to said external annular diaphragm portion 12.

**.** In accordance with an embodiment, said diaphragm 8 comprises an external annular diaphragm portion 12 directly or indirectly connected to said support structure 2.

**.** Each occluding bridge end 51, 52 is in a single piece with said external annular diaphragm portion 12.

**.** In accordance with an embodiment, said diaphragm 8 comprises an external annular diaphragm portion 12 directly or indirectly connected to said support structure 2.

**.** Said diaphragm 8 comprises a central disc-shaped diaphragm portion 20.

**.** Said plurality of occluding bridges 21 is arranged radially with an external end 51 connected to said external annular diaphragm portion 12 and the internal end 52 connected to said central diaphragm disc 20.

**.** In accordance with an embodiment, said diaphragm 8 comprises an external annular diaphragm portion 12 directly or indirectly connected to said support structure 2.

**.** Said diaphragm 8 comprises a central disc-shaped diaphragm portion 20.

**.** Said plurality of occluding bridges 21 is arranged radially with an external end 51 connected to said external annular diaphragm portion 12 and the internal end 52 connected to said central diaphragm disc 20.

**.** The opposite occluding bridge edges or sides 22 converge towards said central diaphragm disc 20; and where each occluding bridge edge or side 22 is arranged parallel to the adjacent occluding bridge edge or side 22 of the adjacent occluding bridge 21.

**.** In accordance with an embodiment, said diaphragm 8 comprises a plurality of hollow tubes or wires or elastic threads 42 comprising hollow tube or elastic thread stretches 41 which form said diaphragm 8.

**.** In accordance with an embodiment, said diaphragm 8 comprises a single hollow tube or wire or elastic thread 40 folded to form hollow tube or wire or elastic thread stretches 41 which form said diaphragm 8.

**.** In accordance with an embodiment, said plurality of hollow tubes or wires or elastic threads 42 or said single folded hollow tube or wire or elastic thread 40 is connected or folded around said support structure 2.

**.** In accordance with an embodiment, said plurality of hollow tubes or wires or elastic threads 42 or said single folded hollow tube or wire or elastic thread 40 is connected or folded around said central support structure portion 3.

**.** In accordance with an embodiment, said diaphragm 8 comprises a plurality of hollow tube or wire or elastic thread stretches 41 which are parallel to one another.

**.** In accordance with an embodiment, said diaphragm 8 comprises a plurality of hollow tube or wire or elastic thread stretches 41 which are mutually arranged side by side.

**.** In accordance with an embodiment, said diaphragm 8 comprises a plurality of hollow tube or wire or elastic thread stretches 41 which are arranged adjacent to one another.

**.** In accordance with an embodiment, said diaphragm 8 comprises a plurality of radially arranged hollow tube or wire or elastic thread stretches 41.

**.** In accordance with an embodiment, said diaphragm 8 comprises a first plurality of hollow tube or wire or elastic thread stretches 41 which are parallel to one another and a second plurality of hollow tube or wire or elastic thread stretches 41 which, in said second plurality, are parallel to one another but orthogonal to said first plurality of hollow tube or wire or elastic thread stretches 41.

**.** In accordance with an embodiment, said diaphragm 8 comprises a first plurality of hollow tube or wire or elastic thread stretches 41 which are parallel to one another and at least a second plurality of hollow tube or wire or elastic thread stretches 41 which, in said at least a second plurality, are parallel to one another but inclined with respect to said first plurality of hollow tube or wire or elastic thread stretches 41.

**.** In accordance with an embodiment, said plurality of hollow tubes or wires or elastic threads 42 or said single hollow tube or wire or elastic thread 40 is made of a superelastic material, preferably Nitinol, or silicone, or polyurethane or medical elastomer or of a bioerodable material.

**.** In accordance with an embodiment, said device comprises at least two diaphragms 8.

**.** Said at least two diaphragms 8 at least partially overlap with each other.

**.** In accordance with an embodiment, said device comprises at least two diaphragms 8.

**.** Said at least two diaphragms 8 at least partially overlap with each other so as to at least partially face at least one occluding bridge 21 of a first diaphragm 8 to at least one elongated and narrow opening or slit or cut 27 of a second diaphragm 8.

**.** In accordance with an embodiment, said device comprises at least two diaphragms 8.

**.** Said at least two diaphragms 8 at least partially overlap with each other so as to at least partially enter an elongated and narrow opening or slit or cut 27 of a second diaphragm 8 with at least one occluding bridge 21 of a first diaphragm 8, by arranging itself between two adjacent occluding bridges 21 of said second diaphragm 8.

**.** In accordance with an embodiment, said support structure 2 is in a single piece. In accordance with an embodiment, said support structure 2 and said diaphragm 8 are in a single piece.

**.** In accordance with an embodiment, said support structure 2 is made of medical elastomer.

**.** In accordance with an embodiment, two diaphragms 8 are included, and where said two diaphragms 8 angularly overlap with each other by arranging the occluding bridges 21 of a first diaphragm 8 crossed with respect to the occluding bridges 21 of the other diaphragm 8.

**.** In accordance with an embodiment, two diaphragms 8 are included; and where said two diaphragms 8 overlap with each other, the occluding bridges 21 of a first diaphragm 8 are arranged inclined with respect to the occluding bridges 21 of the second diaphragm 8.

**.** In accordance with an embodiment, two diaphragms 8 are included; and where said two diaphragms 8 angularly overlap with each other by arranging the occluding bridges 21 of a first diaphragm 8 so as to be orthogonal to the occluding bridges 21 of the other diaphragm 8.

**.** In accordance with an embodiment, two diaphragms 8 are included; and where said two diaphragms 8 angularly overlap with each other by arranging the occluding bridges 21 of a first diaphragm 8 at an angle between 20 degrees and 90 degrees with respect to the occluding bridges 21 of the other diaphragm 8.

. In accordance with an embodiment, said device 1 comprises a diaphragm 8 with occluding bridges 21 in a thread; said occluding bridges 21 are mounted to frame and said frame is fixed to said support structure 2.

. In accordance with an embodiment, said device 1 comprises a diaphragm 8 with occluding bridges 21 having a wavy occluding bridge body 50.

**.** In accordance with an embodiment, said device 1 comprises a diaphragm 8 with occluding bridges 21 having a wavy occluding bridge body 50, thus allowing each wavy bridge 21 urged by a possible crossing of the diaphragm itself, for example by a medical device, to elongate, and then return to a closed position of the diaphragm 8.

. In accordance with an embodiment, said device 1 comprises a diaphragm 8 with occluding bridges 21 having wavy occluding bridge edges 22, the wavy bridge edge being the side portion of the occluding bridge 21 which delimits said elongated and narrow opening or slit or cut 27, thus allowing each occluding bridge urged by a possible crossing of the diaphragm by a medical device, to elongate, and facilitating, once the device has been removed, the re-closing of the wavy bridge 21 and therefore of the diaphragm 8 itself.

**.** In accordance with an embodiment, when under relaxed conditions, said diaphragm 8 is planar in shape and said occluding bridges 21 are tensioned on said support structure 2.

**.** In accordance with an embodiment, said occluding bridges 21 are slats or strips or foils or wires or hollow tubes.

**.** In accordance with an embodiment, under a relaxed condition of said diaphragm 8, said occluding bridges 21 are separated by slits or cuts 27 in order to avoid material continuity between one bridge and the other.

**.** In accordance with an embodiment, under a relaxed condition of said diaphragm 8 and when the device is not implanted in a human body, said occluding bridges 21 close the majority of said lumen 4.

**.** In accordance with an embodiment, under a relaxed condition of said diaphragm 8, said occluding bridges 21 cover 70-100% of the lumen 4.

**.** In accordance with an embodiment, under a relaxed condition of said diaphragm 8, said occluding bridges 21 are uniformly distributed over the lumen 4.

**.** In accordance with an embodiment, under a relaxed condition of said diaphragm 8, said occluding bridges 21 delimit slits or cuts 27 which leave free passages among said occluding bridges 21 of no more than 25% of said lumen 4.

**.** In accordance with an embodiment, said diaphragm 8 is obtained from a single sheet where said elongated slits 27 are made by laser cutting.

**.** In accordance with an embodiment, said diaphragm 8 is obtained from a single sheet 53 in which said elongated slits 27 are made by cuts.

**.** Said sheet 53 is fixed to the support structure 2 crossing said lumen 4 twice.

**.** In accordance with an embodiment, said occluding bridges 21 are concentric rings which extend along an annular path 16.

**.** Adjacent concentric occluding bridges 21 are connected by means of radial small bridges or cut interruptions 17, 18, 19.

**.** Said radial small bridges or cut interruptions 17, 18, 19 are circumferentially offset between three near concentric occluding bridges 21 so as to avoid a radial alignment of a plurality of radial small bridges or cut interruptions.

**.** The sides 22 of adjacent concentric occluding bridges 21 delimit arched slits or cuts 27 aligned along a circumferential path interrupted by said radial small bridges or cut interruptions 17, 18, 19.

**.** In accordance with an embodiment, said diaphragm 8 comprises an external continuous annular diaphragm portion 12 directly or indirectly connected to said support structure 2.

**.** The radially outermost occluding bridge 21 is connected to said external continuous annular diaphragm portion 12 via said radial small bridges or cut interruptions 17, 18, 19.

**.** In accordance with a general embodiment, a crossable interseptal occluder device 1 comprises a support structure 2.

**.** Said support structure 2 comprises a central support structure portion 3 which delimits a lumen 4.

**.** Said support structure 2 comprises a first anchoring portion 5 and an opposite second anchoring portion 6.

**.** Said support structure 2 is configured to expand and contract between a compressed tubular configuration for the insertion through the patient's vasculature and an expanded configuration in which the first and second anchoring portions 5, 6 extend radially outwards from said central support structure portion 3 to compress a partition 7 therebetween by arranging said support structure 2 astride said partition 7 through a defect or hole or opening present in the partition 7.

**.** Said crossable interseptal occluder device 1 further comprises a diaphragm 8.

**.** Said diaphragm 8 is supported by said support structure 2 and arranged to close at least partially said lumen 4.

**.** Said diaphragm 8 being configured to allow a medical instrument inserted into a first compartment 9 or 10 delimited by said septum 7 to pass through the diaphragm 8 and then through said lumen 4 entering a second compartment 10 or 9 delimited by said septum 7.

**.** Said diaphragm 8 comprises a diaphragm edge 11 placed close to said central support structure portion 3 of said support structure 2.

**.** Advantageously, said diaphragm 8 comprises at least one elongated and narrow opening 27.

**.** The term "elongated and narrow opening" means a longitudinally extending opening, predominantly with respect to the extension thereof which is transverse to said longitudinal direction.

**.** Alternatively, said elongated and narrow opening 27 comprises one of the following embodiments:
- it extends from a first diaphragm portion 12 of said diaphragm 8 placed close to a first edge stretch 13 of said diaphragm edge 11, to a second diaphragm portion 14 of said diaphragm 8 placed close to a second edge stretch 15 of said diaphragm edge 11;
   or
- it extends from a first diaphragm portion 12 of said diaphragm 8 placed close to a first edge stretch 13 of said diaphragm edge 11, to a second diaphragm portion 14 of said diaphragm 8 placed close to a diaphragm center 36 of said diaphragm 8;
   or
- it extends close to said diaphragm edge 11 to cover an annular path 16 interrupted by small diaphragm bridges 17, 18, 19 which connect a central diaphragm portion 20 to said diaphragm edge 11 by forming a plurality of elongated and narrow openings 11 placed one after the other.

**.** In accordance with an alternative embodiment, said at least one elongated and narrow opening 27 is a plurality of long and narrow openings 11 delimited by a plurality of elongated membrane fringes 21, or membrane fringes 21 or occluding bridges 21.

**.** In accordance with an alternative embodiment, in said diaphragm 11 said multiple membrane fringes 21 are parallel to one another.

**.** In accordance with an alternative embodiment, in said diaphragm 11 said multiple membrane fringes 21 are mutually placed side by side and spaced apart from said plurality of long and narrow openings 11.

**.** In accordance with an alternative embodiment, said plurality of membrane fringes 21 comprises membrane fringe edges 22 and where the membrane fringe edges 22 of adjacent membrane fringes 21 are mutually placed side by side and in contact with one other so that said elongated and narrow opening 27 is a slit.

**.** In accordance with an alternative embodiment, said plurality of membrane fringes 21 comprises membrane fringe edges 22 and where the membrane fringe edges 22 of adjacent membrane fringes 21 are separated from one another so that said elongated and narrow opening 27 is a free opening.

**.** In accordance with an alternative embodiment, said diaphragm 8 is made of an elastic material, for example silicone or medical elastomer or polyurethane or of a bioerodable material.

**.** In accordance with an alternative embodiment, said diaphragm 8 is a membrane.

**.** In accordance with an alternative embodiment, said diaphragm 8 is an elastic membrane.

**.** In accordance with an alternative embodiment, said membrane fringes 21 of said diaphragm 8 are separated from one another in the extension thereof or central fringe portion 29 but joined together in a single piece.

**.** In accordance with an alternative embodiment, said diaphragm edge 11 and said membrane fringes 21 are in a single piece.

**.** In accordance with an alternative embodiment, each of said membrane fringes 21 has a longitudinal extension and comprises a fringe section 23 evaluated in a transverse direction to said longitudinal direction of square or rectangular shape.

**.** In accordance with an alternative embodiment, each of said membrane fringes 21 has a longitudinal extension and comprises a fringe section 23 evaluated in a transverse direction to said longitudinal direction of tapered shape passing from the center of said section to a section end 24 thereof.

**.** In accordance with an alternative embodiment, each of said membrane fringes 21 has a longitudinal extension and comprises a fringe section 23 evaluated in a transverse direction to said longitudinal direction of tapered shape passing from a first section base side 25 to a section end 24.

**.** In accordance with an alternative embodiment, each of said membrane fringes 21 has a longitudinal extension and comprises a fringe section 23 evaluated in a transverse direction to said longitudinal direction of tapered shape passing from a first section base side 25 to a smaller section side 26.

**.** In accordance with an alternative embodiment, each of said membrane fringes 21 has a longitudinal extension and comprises a fringe section 23 evaluated in a transverse direction to said longitudinal direction of oval or triangular or rhomboidal or trapezoidal shape.

**.** In accordance with an alternative embodiment, a pair of diaphragms 28 is included, each of said diaphragms 8 of said pair of diaphragms 28 comprising a plurality of membrane fringes 21 which delimit a plurality of elongated and narrow openings 27.

**.** Said diaphragms 8 of said pair of diaphragms 28 are arranged facing each other and with the plurality of membrane fringes 21 of the first diaphragm 8 of said pair of diaphragms 28 being offset with respect to the plurality of membrane fringes 21 of the second diaphragm 8 of said pair of diaphragms 28 by overlapping at least partially said plurality of membrane fringes 21 of the first diaphragm 8 to said plurality of elongated and narrow openings 27 of said second diaphragm 8.

**.** In accordance with an alternative embodiment, said plurality of membrane fringes 21 of the first diaphragm 8 has tapered fringe sections 23.

**.** In accordance with an alternative embodiment, said plurality of membrane fringes 21 of the first diaphragm 8 has tapered fringe sections 23, for example with rhomboid- or triangle-shaped section.

**.** In accordance with an alternative embodiment, the tapering of the fringe sections 23 of said plurality of membrane fringes 21 of the first diaphragm 8 tapers towards said second diaphragm 8.

**.** In accordance with an alternative embodiment, said plurality of membrane fringes 21 of the first diaphragm 8 interpenetrates at least partially between said membrane fringes 21 of said plurality of membrane fringes 21 of the second diaphragm 8.

**.** In accordance with an alternative embodiment, said diaphragm 8 has a concave body or concavity facing one of said first or second compartment 9, 10.

**.** In accordance with an alternative embodiment, said diaphragm 8 is dome-shaped.

**.** In accordance with an alternative embodiment, said diaphragm 8 comprises a diaphragm stiffening structure 30.

**.** In accordance with an alternative embodiment, said diaphragm stiffening structure 30 comprises diaphragm ribs 31 adapted to create a support scaffold.

**.** In accordance with an alternative embodiment, said diaphragm stiffening structure 30 comprises diaphragm ribs 31 in a single piece with said diaphragm 8.

**.** In accordance with an alternative embodiment, said diaphragm stiffening structure 30 comprises at least one diametrical rib 32 extending along a diameter of said diaphragm 8.

. In accordance with an alternative embodiment, said diaphragm stiffening structure 30 comprises at least one circumferentially extending rib 33.

**.** In accordance with an alternative embodiment, said diaphragm stiffening structure 30 comprises at least one radially extending rib 34.

**.** In accordance with an embodiment, said at least one radially extending rib is made of an extendable, i.e. stretchable, elastic material.

**.** In accordance with an alternative embodiment, said diaphragm stiffening structure 30 comprises at least one rib 35 extending along a circle chord.

**.** In accordance with an embodiment, said at least one rib extending along a circle chord is made of an extendable, i.e. stretchable, elastic material.

**.** In accordance with an alternative embodiment, said diaphragm 8 comprises at least one diaphragm portion shaped as a diaphragm half-dome 38, where said diaphragm half-dome 38 is separated from one diametrical rib 32 of one diaphragm stiffening structure 30 by an elongated and narrow opening 27 shaped as an arch.

**.** In accordance with an alternative embodiment, said elongated and narrow opening 27 shaped as an arch extends from a first diaphragm portion 12 of said diaphragm 8 placed close to a first edge stretch 13 of said diaphragm edge 11, to a second diaphragm portion 14 of said diaphragm 8 placed close to a second edge stretch 15 of said diaphragm edge 11.

**.** In accordance with an alternative embodiment, said diaphragm 8 comprises two diaphragm portions shaped as a diaphragm half-dome 38, where said diaphragm half-domes 38 are mutually placed side by side to form a diaphragm dome 39 and each of said diaphragm half-domes 38 is separated from a diametrical rib 32 of a diaphragm stiffening structure 30 by an elongated and narrow opening 27 shaped as an arch.

**.** In accordance with an alternative embodiment, said elongated and narrow opening 27 shaped as an arch of each of said two diaphragm half-domes 38 extends from a first diaphragm portion 12 of said diaphragm 8 placed close to a first edge stretch 13 of said diaphragm edge 11, to a second diaphragm portion 14 of said diaphragm 8 placed close to a second edge stretch 15 of said diaphragm edge 11.

**.** In accordance with an alternative embodiment, said diaphragm 8 comprises a plurality of elastic threads 42 comprising elastic thread stretches 41 which form said diaphragm 8.

**.** In accordance with an alternative embodiment, said diaphragm 8 comprises a single elastic thread 40 folded to form elastic thread stretches 41 which form said diaphragm 8.

**.** In accordance with an alternative embodiment, said plurality of elastic threads 42 or said single folded elastic thread 40 is connected or folded around said support structure 2.

**.** In accordance with an alternative embodiment, said plurality of elastic threads 42 or said single folded elastic thread 40 is connected or folded around said central support structure portion 3.

**.** In accordance with an alternative embodiment, said diaphragm 8 comprises a plurality of elastic thread stretches 41 which are parallel to one another.

**.** In accordance with an alternative embodiment, said diaphragm 8 comprises a plurality of elastic thread stretches 41 which are mutually arranged side by side.

**.** In accordance with an alternative embodiment, said diaphragm 8 comprises a plurality of elastic thread stretches 41 which are arranged adjacent to one another.

**.** In accordance with an alternative embodiment, said diaphragm 8 comprises a plurality of radially arranged elastic thread stretches 41.

**.** In accordance with an alternative embodiment, said diaphragm 8 comprises a first plurality of elastic thread stretches 41 which are parallel to one another and a second plurality of elastic thread stretches 41 which, in said second plurality, are parallel to one another but orthogonal to said first plurality of elastic thread stretches 41.

**.** In accordance with an alternative embodiment, said diaphragm 8 comprises a first plurality of elastic thread stretches 41 which are parallel to one another and at least a second plurality of elastic thread stretches 41 which, in said at least a second plurality, are parallel to one another but inclined with respect to said first plurality of elastic thread stretches 41.

**.** In accordance with an alternative embodiment, said plurality of elastic threads 42 or said single elastic thread 40 is made of a superelastic material, preferably Nitinol, or silicone, or polyurethane or medical elastomer or of a bioerodable material.

**.** In accordance with an alternative embodiment, said device comprises at least two diaphragms 8.

**.** In accordance with an alternative embodiment, said at least two diaphragms 8 at least partially overlap with each other.

**.** In accordance with an alternative embodiment, said at least two diaphragms 8 at least partially overlap with each other so as to at least partially face a membrane fringe 21 of a first diaphragm 8 to an elongated and narrow opening 27 of a second diaphragm 8.

**.** In accordance with an alternative embodiment, said at least two diaphragms 8 at least partially overlap with each other so as to at least partially enter an elongated and narrow opening 27 of a second diaphragm 8 with at least one membrane fringe 21 of a first diaphragm 8, by arranging itself between two adjacent membrane fringes 21 of said second diaphragm 8.

**.** In accordance with an alternative embodiment, said support structure 2 is in a single piece.

**.** In accordance with an alternative embodiment, said support structure 2 and said diaphragm 8 are in a single piece.

**.** In accordance with an alternative embodiment, said support structure 2 is made of medical elastomer.

**.** Those skilled in the art can make several changes and adaptations to the embodiments described above, and replace elements with others which are functionally equivalent, in order to meet contingent and specific needs, without however departing from the scope of the following claims.

**.** Some exemplary embodiments of the present invention will be described below.

**.** In accordance with an embodiment, the device 1 is made to be implanted by means of minimally invasive or percutaneous techniques.

**.** One of the peculiarities of the device 1 consists in the possibility of being crossed again after some time from its implantation, in order to start new interseptal procedures.

. In accordance with an embodiment, the architecture of the device 1 is based on two components or parts:
- a frame, or support structure 2, dedicated to the positioning and anchoring in situ of the device, generally toroidal in shape with opposing "umbrellas" or "donuts", positioned astride the defect or hole or opening, with the umbrellas or donuts arranged on opposite sides of the partition 100;
- a diaphragm 8, for example a central diaphragm, dedicated to the occlusion of the defect/hole, for example an iatrogenic or congenital defect/hole 107, with the possibility of being crossed again after some time.

**.** The support structure 2 can be identified among those currently used or in any case described in the prior art, or it can be conceived and implemented according to an embodiment as previously described here.

**.** From the construction point of view, for example, two main types of solutions can be referred to:
- support structure 2 obtained by means of techniques of braiding metal wires made of superelastic alloy (e.g. Nitinol; CoCr);
- support structure 2 obtained from a laser-cut and then shaped metal tube made of superelastic alloy.

**.** For the diaphragm 8, or occluding diaphragm, some exemplary embodiments are described below.
1) Diaphragm 8 with occluding bridges in an "elastic fringe curtain".

**.** This diaphragm 8 consists of thin strips (fringes 21) of elastic and highly extendable material. The fringes 21 are parallel and adjacent to one another, or with a small gap to space them apart, or arranged in a sunburst pattern, or according to other construction schemes. The diaphragm 8 can also consist of one or more layers of variously crossed fringes 21. Upon crossing by the catheter, the fringes 21 move apart and elongate, thus allowing the re-crossing. The re-crossing point can be freely decided by the operator at any point of the diaphragm 8. Advantageously, the suggested solution for diaphragm and fringes, after the removal of the device, allows to re-close the fringes 21 and therefore the diaphragm 8 itself.

### . 1-a) diaphragm with occluding bridges in an "elastic fringe curtain" in the shape of a thin disc

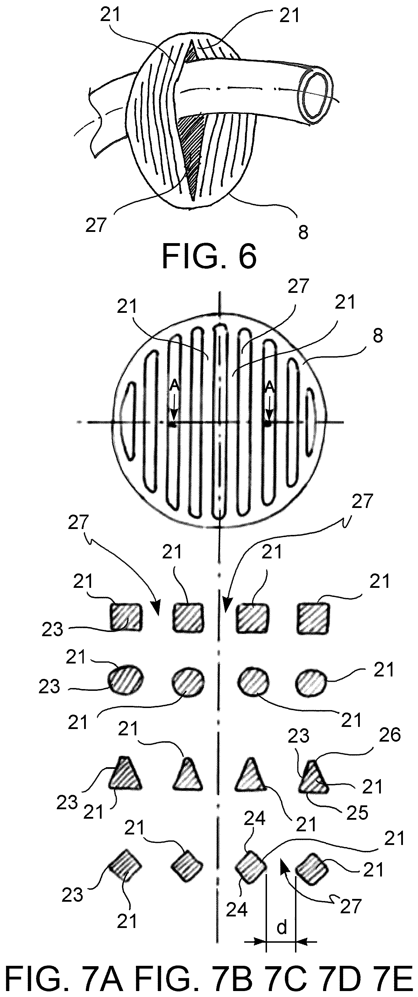

**.** Depending on the technology adopted for the manufacture, the elastic fringes 21, and in particular the cross sections thereof, can be made in various shapes, as in the example in figures 7B to 7E, 8A and 8B, and 9.

**.** The distance "d", shown in Figure 7E, between the fringes 21 can vary between zero (for example in diaphragms obtained from cut membranes) and a distance of the order of one millimeter. Distances of such an extent, indeed, are destined to be soon occluded by natural tissue regrowth.

**.** The individual fringes 21 can have different shapes and orientation, and the distance therebetween may also be nonhomogeneous.

**.** In terms of materials, thin-disc diaphragms 8 can typically be made of silicone or polyurethane, choosing the types the physical-mechanical features of which are most suitable for the intended use.

**.** In technological terms, the diaphragms 8 with elastic fringes 21 can be obtained:
- from membranes then incised in the form of a fringe;
- by molding;
- by means of 3-D printing processes;
- by means of subsequent operations of gluing various components.

**.** The diaphragm 8 in the shape of a thin disc can also be made from bioabsorbable polymers. In this case, the possibility of re-crossing will typically be obtained at the end of the bio-absorption process, due to the substantial replacement of the diaphragm 8 with the patient's natural tissue. By adopting particularly elastic bio-absorbable polymers, re-crossability will be ensured, immediately after implantation, by an operating mechanism which is similar to that of the silicone or polyurethane diaphragms (offset + elongation of fringes).

**.** As regards the fixing of the diaphragm 8, for example in the center of the support structure 2, this can be done according to one of the techniques listed here:
- gluing
- heat sealing
- stitching
- co-molding
- crimping
- interlocking
- a mixed solution among the previous ones

**.** 1-b) diaphragm with occluding bridges in an "elastic fringe curtain" obtained with elastic threads.

**.** A second embodiment for diaphragms 8 with elastic fringes 21 is that which can be obtained from elastic and highly extendable threads 40. Such a solution can be implemented ac-cording to various approaches. For example, in a first solution, the threads 40 may be applied directly to the structures of the support structure. Or, in a second example, the threads 40 may be applied to a frame, in turn mounted to the support structure.

**.** The threads 40 may be applied so as to create different patterns: parallel, crossed, radial threads.

**.** In terms of materials, the same materials listed for the thin diss solution, including bioerodible polymers, are suitable for these threads 40.

**.** The threads 40 can be obtained by extrusion or other known techniques and applied to the frames or directly to the support structure 2 by:
- winding
- binding
- heat sealing
- gluing
- pinching
- other known tech-niques
- mixed solutions among those listed

### . 1-c) diaphragm with occluding bridges in a "dome of elastic fringes"

**.** Physiologically, pressure differences exist between the two sides of the atrial partition, which vary within the cardiac cycle. In particular, the pressure in left atrium 102 is higher than that in the right atrium 101. A constructional solution aimed at minimizing the passage of blood from the chamber, or compartment, at a higher pressure to that at a lower pressure can be that of the "dome of elastic fringes".

**.** The solution essentially follows that of the disc of elastic fringes described in item 1-a but, instead of developing flat, it precisely develops in the shape of a dome. The dome will be mounted to the support structure with the convexity facing the higher pressure chamber. Thereby, the blood itself, at a higher pressure, will tend to compact the adjacent fringes 21 together, thus increasing the tightness of the occlusion.

**.** As for the materials, the technologies for making the dome, and how to fix it to the frame, the same related to the disc solution described above applies.

**.** Some exemplary embodiment of an occluding device 1 according to the present invention will be described below.

**.** In particular, the solution described herein has an "integral solution", or in a single piece, (support structure 2 + diaphragm 8) made of medical elastomer, for example silicone or polyurethane.

**.** In general terms, the description of the device coincides with that given in the previous embodiments, taking into account that in this embodiment the support structure does not derive from known solutions made of metal alloy, but is made of medical elastomer.

**.** The advantages of this solution compared to those already described mainly lie in the high simplicity and cost-effectiveness of the construction, as well as in a greater lightness compared to the traditional occluders obtained from metal alloys.

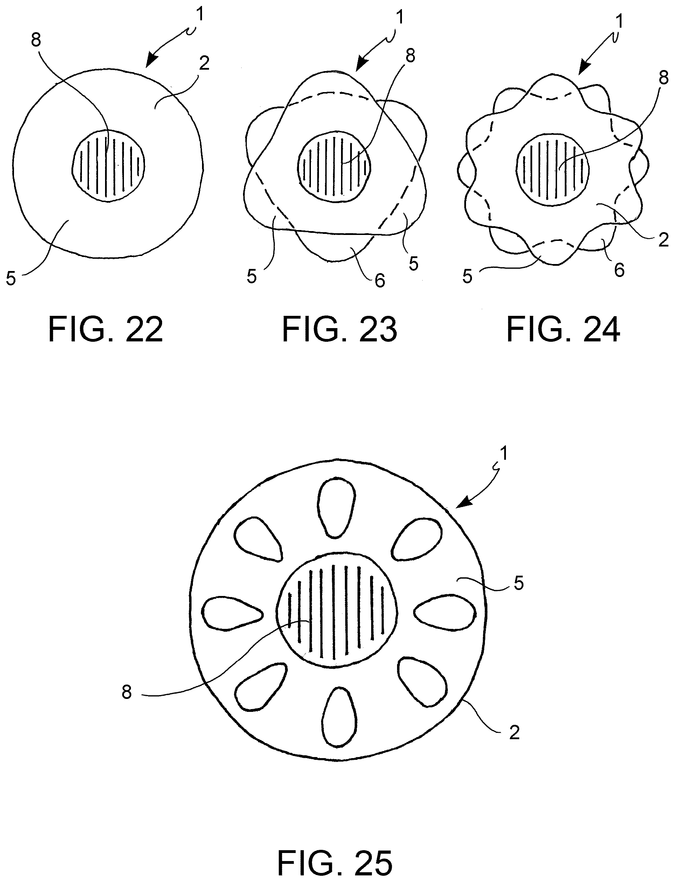

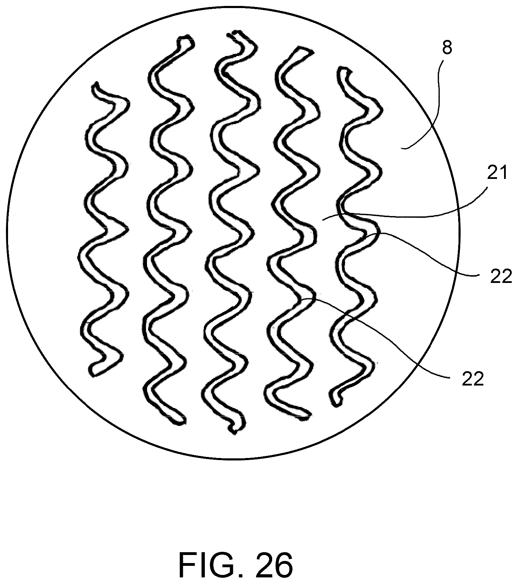

**.** In Figures 22 to 30, the diaphragm 8 is depicted in its shape of a "curtain of elastic fringes", but it will be able to be made according to any of the solutions described above.

**.** The general structure of the device reflects that already described, which includes:
- a support structure 2 dedicated to the positioning and anchoring in situ of the device 1, generally toroidal in shape with opposing "umbrellas" or "donuts", positioned astride the defect or hole, with the umbrellas or donuts arranged on opposite sides of the interatrial partition;
- a diaphragm 8 or central diaphragm dedicated to the occlusion of the iatrogenic or congenital defect/hole, with the possibility of being crossed again after some time.

**.** In this case, the support structure 2 is made of medical elastomer, and is characterized by a specific design, adapted to utilize the features of that family of materials and to optimize the functionality of the device:
- compression along the ridge;
- convexity facing the atrial chamber.

**.** The support structure 2, in accordance with an embodiment, has a convexity facing the atrial chambers. This peculiarity offers a range of advantages:
- a gap is created between the support structure and the interatrial partition. In this gap, the tissue of the atrial partition comes out during the expansion and in-situ release of the device. In fact, it must be considered that the iatrogenic or congenital defect or hole to be occluded will have a typically smaller diameter than that of the occluder, and an irregular shape. Under the radial thrust of the occluder, the excess tissue will widen and deform, and will be accommodated in the gap provided between the anchoring portions. In the absence of this gap, the excess tissue could hinder the regular expansion of the occluder device 1;
- due to the shape provided, the support structure 2 rests on the interatrial partition mainly along the external edge only, concentrating along such an edge the compression force which stabilizes the device 1 in place. Thereby, the contact pressure is high, and the adhesion of the support structure 2 to the partition is optimal.

**.** The above effects can be modulated by acting on dimensions and other measures as shown in Figures 23 to 26.

**.** In particular, the thickening of the edge (Figure 24), or the inclusion of a reinforcing material (Figure 25), can strongly increase the resistance to displacement.

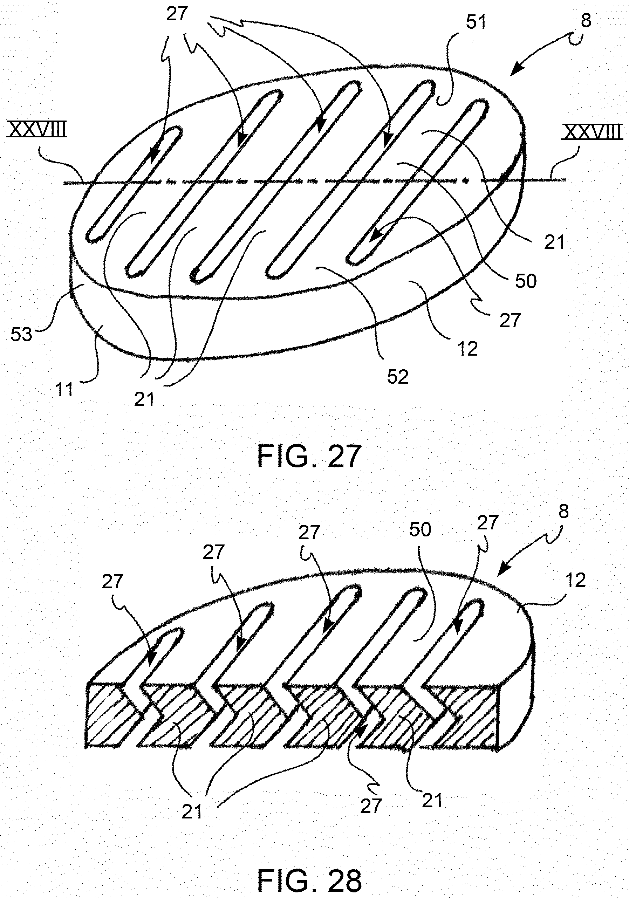

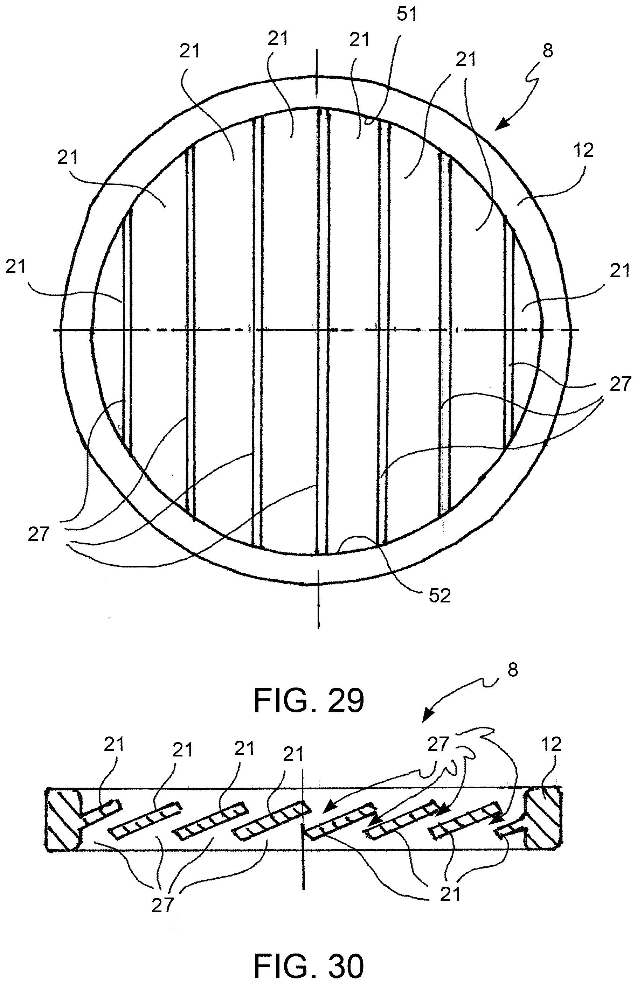

**.** Even in terms of the general shape of the support structure, it is possible to manufacture the device according to various solutions, according to the desired performance. For example, lobed forms for the frame umbrellas, with lobes staggered between the two sides of the partition, can facilitate the anchoring and the ease of "crimping" the device on a delivery catheter (Figures 28 and 29).

**.** It is also possible to vary the design of the umbrellas by making cuts or openings in appropriate shapes and positions. Thereby, the device 1 is lightened, the creation of massive thrombotic formations between the umbrella and the interatrial partition is avoided, and the "crimping" of the device on the delivery catheter is facilitated (Figure 30).

**.** In terms of materials, the whole device 1 is made of medical elastomer, typically silicone or polyurethane. Alternatively, the whole device 1 is made of bio-erodible polymers.

**.** The preferred solution is obtained by molding, according to known techniques, the component in a single piece.

**.** In accordance with an embodiment, the device can also be obtained by assembling, typically by gluing, parts obtained with elastomers which are different in hardness or elasticity. For example, the support structure 2 could be made of a single piece of more rigid elastomer, by applying a more pliable elastomer diaphragm 8 in the center.

**.** There is also the possibility of co-molding, inserting, applying other local reinforcement components, or intended for other functions, made of different materials: metallic alloys, polymer threads, fabrics, and the like.

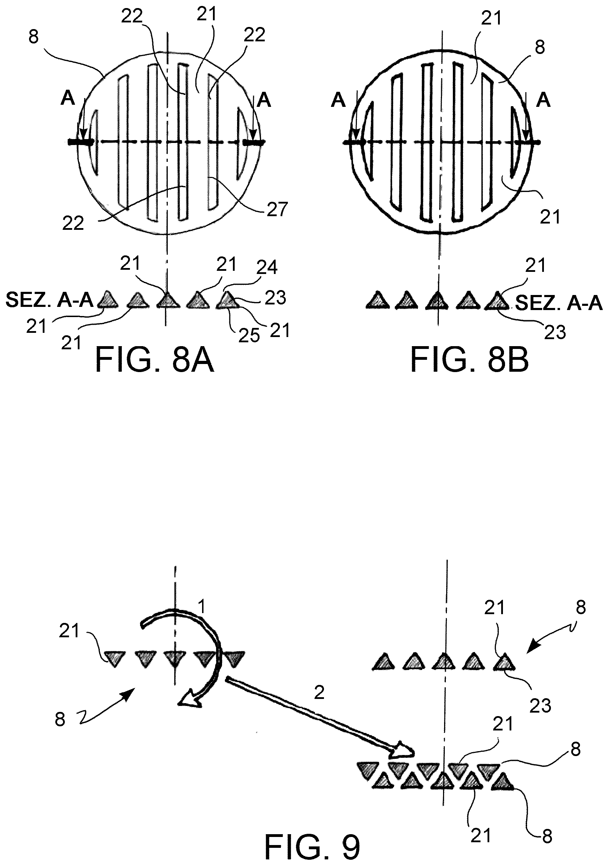

**.** In accordance with an alternative embodiment, two fringed diaphragms 8 are provided, the fringes 21 of which have a tapered cross section, for example triangular or trapezoidal. Said two diaphragms 8 overlap with each other and are slightly staggered, so as to intercalate the respective fringes 21 and arrange the triangular or rhomboidal sections of either diaphragm at least partially interpenetrated to improve the fluid tightness, as shown in Figures 8A, 8B, 9, for example.

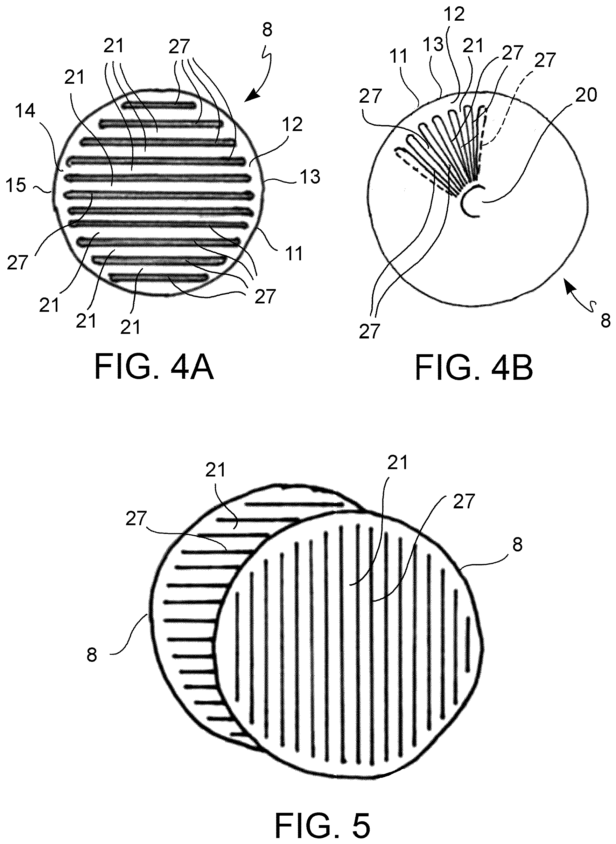

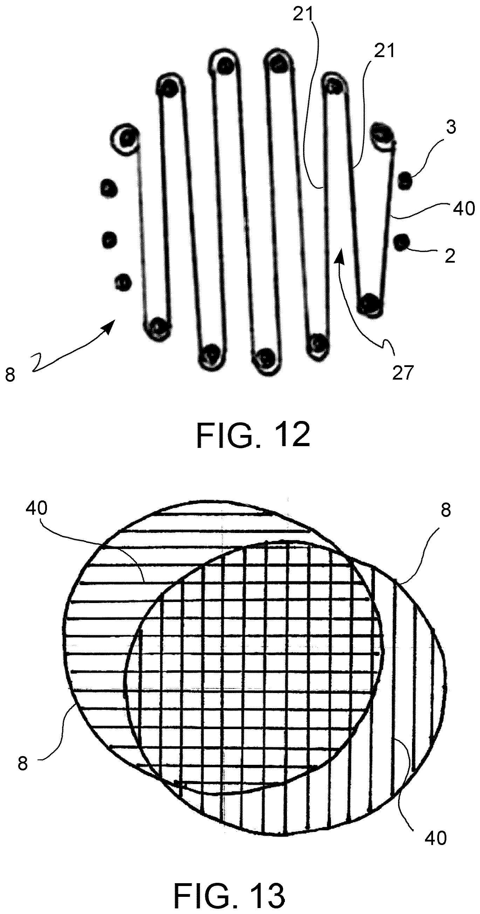

**.** In accordance with an alternative embodiment, two fringed diaphragms 8 are provided. Said two fringed diaphragms 8 overlap with each other by angularly arranging the fringes 21 of a diaphragm 8 crossed with respect to the fringes 21 of the other diaphragm 8. For example, the fringes 21 of a first diaphragm 8 are arranged orthogonal to the fringes 21 of the second diaphragm 8, as shown in Figure 5 or in Figure 13, for example, where the fringes are in a thread.

**.** In accordance with an alternative embodiment, a diaphragm 8 with fringes 21 in a thread is provided. Said thread-like fringes 21 are mounted to frame and said frame is fixed to said support structure 2.

**.** In accordance with an alternative embodiment, a diaphragm 8 with fringes 21 having a wavy fringe body is provided, thus allowing each fringe urged by a possible crossing of the diaphragm, to elongate, and then return to a closed position of the diaphragm 8 itself.

**.** In accordance with an alternative embodiment, a diaphragm 8 with fringes 21 having wavy fringe edges is provided, the fringe edge being the lateral portion of the fringe which delimits said elongated and narrow opening 27, thus allowing each fringe urged by a possible crossing of the diaphragm by a medical device, to elongate, but facilitating, once the device has been removed, the re-closing of the fringes 21 and therefore of the diaphragm 8.

**.** All different embodiments of diaphragm 8 and fringes 21 thereof described above advantageously allow the re-crossing of the diaphragm 8, after its implantation, and equally advantageously, after the removal of said medical device, allow the re-closing of the fringes 21 and therefore the re-closing of the diaphragm 8 itself.

### LIST OF REFERENCE NUMERALS

- 1: crossable interseptal occluder device
- 2: support structure
- 3: central support structure portion
- 4: lumen
- 5: first anchoring portion
- 6: second anchoring portion
- 7: partition
- 8: diaphragm
- 9: first compartment
- 10: second compartment
- 11: diaphragm edge
- 12: first diaphragm portion or external continuous annular diaphragm portion
- 13: first edge stretch
- 14: second diaphragm portion
- 15: second edge stretch
- 16: annular path
- 17: small diaphragm bridges
- 18: small diaphragm bridges
- 19: small diaphragm bridges
- 20: central diaphragm portion
- 21: elongated membrane fringes or occluding bridges
- 22: membrane fringe edges or occluding bridge edges or occluding bridge sides
- 23: fringe section or bridge section
- 24: section end
- 25: first section base side
- 26: smaller section side
- 27: elongated and narrow opening or cut
- 28: pair of diaphragms
- 29: central fringe portion or central occluding bridge portion
- 30: diaphragm stiffening structure
- 31: diaphragm ribs
- 32: diametrical rib
- 33: circumferentially extending rib
- 34: radially extending rib
- 35: rib extending along a circle chord
- 36: diaphragm center
- 38: diaphragm half-dome
- 39: diaphragm dome
- 40: elastic threads or single elastic thread
- 41: elastic thread stretches
- 42: plurality of elastic threads
- 50: occluding bridge body
- 51: occluding bridge body end
- 52: occluding bridge body end
- 53: single sheet forming said diaphragm
- 54: first sheet edge
- 55: second sheet edge
- 100: atrial partition
- 101: right atrium
- 102: left atrium
- 103: heart
- 104: ventricular partition
- 105: right ventricle
- 106: left ventricle
- 107: defect or opening

## Claims

1. A crossable interseptal occluder device (1) comprising:
a support structure (2); wherein
said support structure (2) comprises a central support structure portion (3) which delimits a lumen (4);
said support structure (2) comprises a first anchoring portion (5) and an opposite second anchoring portion (6);
said support structure (2) being configured to expand and contract between a compressed tubular configuration for insertion through a patient's vasculature, and an expanded configuration, in which the first and second anchoring portions (5, 6) extend radially outwards from said central support structure portion (3) to compress a partition (7) therebetween arranging said support structure (2) astride said partition (7) through a defect or hole or opening present in the partition (7);
and wherein
said crossable interseptal occluder device (1) further comprises a diaphragm (8) made of elastic material capable of a deformation of at least 150%;
said diaphragm (8) being supported by said support structure (2) and arranged to occlude a majority of said lumen (4) when said diaphragm (8) is in a relaxed configuration;
said diaphragm (8) comprising a plurality of slits (27); and
said diaphragm (8) being configured to allow a medical instrument inserted into a first compartment (9 or 10) delimited by said partition (7) to pass through the diaphragm (8) via any one slit (27) of said plurality of slits (27) and then through said lumen (4) entering a second compartment (10 or 9) delimited by said partition (7); wherein
said diaphragm (8) comprises a plurality of occluding bridges (21);
each of said plurality of occluding bridges (21) comprises an elongated body (50) having opposite elongated body ends (51, 52) and opposite longitudinal edges (22);
in the relaxed configuration of said diaphragm (8), each occluding bridge (21) of said plurality of occluding bridges (21) is disposed adjacent to at least a further occluding bridge (21) of said plurality of occluding bridges (21);
at least a portion of said longitudinal edges (22) of any occluding bridge (21) and at least a portion of said longitudinal edge (22) of said further occluding bridge (21) delimiting at least a slit of the plurality of slits (27);
both said opposite ends (51, 52) of each occluding bridge (21) are directly or indirectly connected to or supported by said support structure (2).

2. The crossable interseptal occluder device (1) according to claim 1, wherein in the relaxed configuration of said diaphragm (8), each slit of said plurality of slits (27) has a longitudinal extension equal to an entire longitudinal extension of said elongated body (50) of each occluding bridge (21) delimiting said slit (27).

3. The crossable interseptal occluder device (1) according to any of the preceding claims, wherein said longitudinal edges (22) of adjacent occluding bridges (21) are side by side and in contact with one another.

4. The crossable interseptal occluder device (1) according to any of the preceding claims, wherein in the relaxed configuration of said diaphragm (8), each of said plurality of slits (27) rests in a plane disposed orthogonal to an axis of said lumen (4).

5. The crossable interseptal occluder device (1) according to any of the preceding claims, wherein both of said opposite elongated body ends (51, 52) of each occluding bridge (21) are directly or indirectly connected to or supported by said central support structure portion (3) of said support structure (2).

6. The crossable interseptal occluder device (1) according to any of the preceding claims, wherein said diaphragm (8) is an elastic membrane.

7. The crossable interseptal occluder device (1) according to any of the preceding claims, wherein said diaphragm (8) is configured to prevent a flow of blood through said lumen (4) in the relaxed configuration.

8. The crossable interseptal occluder device (1) according to any of the preceding claims, further comprising a second diaphragm (8), said second diaphragm (8) comprising a second plurality of slits (27), wherein said diaphragm (8) and said second diaphragm (8) are arranged facing each other and with the plurality of slits (27) of the diaphragm (8) offset with respect to the plurality of slits (27) of the second diaphragm (8).

9. The crossable interseptal occluder device (1) according to any of the preceding claims, wherein said diaphragm (8) comprises a plurality of elastic threads (42) which form said diaphragm (8).

10. The crossable interseptal occluder device (1) according to any of claims 1 to 7 or claim 9 when not depending on claim 8, further comprising a second diaphragm (8), said second diaphragm (8) comprising a second plurality of slits (27), wherein said diaphragm (8) and said second diaphragm (8) are arranged facing each other and with the plurality of slits (27) of the diaphragm (8) substantially orthogonal with respect to the plurality of slits (27) of the second diaphragm (8).

11. The crossable interseptal occluder device (1) according to any of the preceding claims, wherein in the relaxed configuration of said diaphragm (8), said plurality of occluding bridges (21) cover 70-100% of the lumen (4).

12. The crossable interseptal occluder device (1) according to any of the preceding claims, wherein in the relaxed configuration, each slit of said plurality of slits (27) has a diameter of between zero millimeters and one millimeter.

13. The crossable interseptal occluder device (1) according to claim 12, wherein said diaphragm (8) is configured to allow natural tissue regrowth to occlude the plurality of slits (27).

14. The crossable interseptal occluder device (1) according to any of the preceding claims, wherein except for the opposite ends (51, 52), each elongated body (50) of each occluding bridge (21) is independent from other elongated bodies (50) of other occluding bridges (21).

15. The crossable interseptal occluder device (1) according to any of the preceding claims, wherein said diaphragm (8) is made of silicone, a medical elastomer, or polyurethane.

## Patentansprüche

1. Durchquerbare interseptale Verschlussvorrichtung (1), umfassend:
eine Stützstruktur (2); wobei
die Stützstruktur (2) einen zentralen Stützstrukturabschnitt (3) umfasst, der ein Lumen (4) begrenzt;
die Stützstruktur (2) einen ersten Verankerungsabschnitt (5) und einen gegenüberliegenden zweiten Verankerungsabschnitt (6) umfasst;
wobei die Stützstruktur (2) dazu konfiguriert ist, sich zwischen einer komprimierten röhrenförmigen Konfiguration zum Einführen durch das Gefäßsystem eines Patienten und einer erweiterten Konfiguration, in der die ersten und zweiten Verankerungsabschnitte (5, 6) sich radial nach außen von dem zentralen Stützstrukturabschnitt (3) erstrecken, um ein Septum (7) dazwischen zu komprimieren, indem die Stützstruktur (2) rittlings auf das Septum (7) durch eine Öffnung oder eine Bohrung oder eine Öffnung, die in dem Septum (7) vorhanden ist, angeordnet wird; und wobei
die durchquerbare interseptale Verschlussvorrichtung (1) ferner eine Membran (8) aus elastischem Material umfasst, die in der Lage ist, eine Verformung von mindestens 150% einzunehmen;
wobei die Membran (8) durch die Stützstruktur (2) getragen wird und so angeordnet ist, dass sie in einer entspannten Konfiguration einen Großteil des Lumens (4) verschließt;
wobei die Membran (8) eine Vielzahl von Schlitzen (27) umfasst; und
wobei die Membran (8) dazu konfiguriert ist, zu erlauben, einem medizinischen Instrument, das in einen ersten Raum (9 oder 10), der durch das Septum (7) begrenzt wird, eingeführt wird, durch die Membran (8) über einen Schlitz (27) der Vielzahl von Schlitzen (27) durchzugehen und dann durch das Lumen (4) in einen zweiten Raum (10 oder 9), der durch das Septum (7) begrenzt wird, einzutreten; wobei
die Membran (8) eine Vielzahl von Verschlussbrücken (21) umfasst;
wobei jede Verschlussbrücke (21) der Vielzahl von Verschlussbrücken (21) einen länglichen Körper (50) aufweisend gegenüberliegende Enden (51, 52) des länglichen Körpers und gegenüberliegende Längsränder (22) umfasst;
wobei in der entspannten Konfiguration der Membran (8) jede Verschlussbrücke (21) der Vielzahl von Verschlussbrücken (21) angrenzend an mindestens einer weiteren Verschlussbrücke (21) der Vielzahl von Verschlussbrücken (21) angeordnet ist;
wobei mindestens ein Abschnitt der Längsränder (22) einer beliebigen Verschlussbrücke (21) und mindestens ein Abschnitt des Längsrandes (22) der weiteren Verschlussbrücke (21) mindestens einen Schlitz der Vielzahl von Schlitzen (27) begrenzen;
wobei beide gegenüberliegenden Enden (51, 52) jeder Verschlussbrücke (21) direkt oder indirekt mit der Stützstruktur (2) verbunden sind oder von der Stützstruktur (2) getragen sind.

2. Durchquerbare interseptale Verschlussvorrichtung (1) nach Anspruch 1, wobei in der entspannten Konfiguration der Membran (8), jeder Schlitz der Vielzahl von Schlitzen (27) eine Längserstreckung aufweist, die der gesamten Längserstreckung des länglichen Körpers (50) jeder Verschlussbrücke (21) gleich ist, der den Schlitz (27) begrenzt.

3. Durchquerbare interseptale Verschlussvorrichtung (1) nach einem der vorhergehenden Ansprüche, wobei die Längsränder (22) angrenzender Verschlussbrücken (21) nebeneinander und in Kontakt miteinander angeordnet sind.

4. Durchquerbare interseptale Verschlussvorrichtung (1) nach einem der vorhergehenden Ansprüche, wobei in der entspannten Konfiguration der Membran (8) jeder der Vielzahl von Schlitzen (27) in einer Ebene liegt, die orthogonal zu einer Achse des Lumens (4) angeordnet ist.

5. Durchquerbare interseptale Verschlussvorrichtung (1) nach einem der vorhergehenden Ansprüche, wobei beide gegenüberliegenden länglichen Körperenden (51, 52) jeder Verschlussbrücke (21) direkt oder indirekt mit dem zentralen Stützstrukturabschnitt (3) der Stützstruktur (2) verbunden sind oder von diesem getragen sind.

6. Durchquerbare interseptale Verschlussvorrichtung (1) nach einem der vorhergehenden Ansprüche, wobei die Membran (8) eine elastische Membran ist.

7. Durchquerbare interseptale Verschlussvorrichtung (1) nach einem der vorhergehenden Ansprüche, wobei die Membran (8) ausgebildet ist, einen Blutfluss durch das Lumen (4) in der entspannten Konfiguration zu verhindern.

8. Durchquerbare interseptale Verschlussvorrichtung (1) nach einem der vorhergehenden Ansprüche, ferner umfassend eine zweite Membran (8), wobei die zweite Membran (8) eine zweite Vielzahl von Schlitzen (27) umfasst, wobei die Membran (8) und die zweite Membran (8) einander gegenüberliegen und mit der Vielzahl von Schlitzen (27) der Membran (8), die versetzt zu der Vielzahl von Schlitzen (27) der zweiten Membran (8) angeordnet ist.

9. Durchquerbare interseptale Verschlussvorrichtung (1) nach einem der vorhergehenden Ansprüche, wobei die Membran (8) eine Vielzahl von elastischen Fäden (42) umfasst, die die Membran (8) bilden.

10. Durchquerbare interseptale Verschlussvorrichtung (1) nach einem der Ansprüche 1 bis 7 oder Anspruch 9, wenn dieser nicht von Anspruch 8 abhängt, ferner umfassend eine zweite Membran (8), wobei die zweite Membran (8) eine zweite Vielzahl von Schlitzen (27) umfasst, wobei die Membran (8) und die zweite Membran (8) einander gegenüberliegen und mit der Vielzahl von Schlitzen (27) der Membran (8), die im Wesentlichen orthogonal zu der Vielzahl von Schlitzen (27) der zweiten Membran (8) angeordnet ist.

11. Durchquerbare interseptale Verschlussvorrichtung (1) nach einem der vorhergehenden Ansprüche, wobei in der entspannten Konfiguration der Membran (8) die Vielzahl von Verschlussbrücken (21) 70-100% des Lumens (4) abdeckt.

12. Durchquerbare interseptale Verschlussvorrichtung (1) nach einem der vorhergehenden Ansprüche, wobei in der entspannten Konfiguration jeder Schlitz der Vielzahl von Schlitzen (27) einen Durchmesser zwischen null Millimetern und einem Millimeter aufweist.

13. Durchquerbare interseptale Verschlussvorrichtung (1) nach Anspruch 12, wobei die Membran (8) ausgebildet ist, einem natürlichen Gewebewachstum zu erlauben, die Vielzahl von Schlitzen (27) zu verschließen.

14. Durchquerbare interseptale Verschlussvorrichtung (1) nach einem der vorhergehenden Ansprüche, wobei mit Ausnahme der gegenüberliegenden Enden (51, 52) jeder längliche Körper (50) jeder Verschlussbrücke (21) unabhängig von anderen länglichen Körpern (50) anderer Verschlussbrücken (21) ist.

15. Durchquerbare interseptale Verschlussvorrichtung (1) nach einem der vorhergehenden Ansprüche, wobei die Membran (8) aus Silikon, einem medizinischen Elastomer oder Polyurethan hergestellt wird.

## Revendications

1. Dispositif d'occlusion interseptale traversable (1) comprenant :
une structure de support (2) ; dans laquelle
ladite structure de support (2) comprend une partie centrale de structure de support (3) qui délimite une lumière (4) ;
ladite structure de support (2) comprend une première partie d'ancrage (5) et une seconde partie d'ancrage opposée (6) ;
ladite structure de support (2) étant configurée pour se dilater et se contracter entre une configuration tubulaire comprimée pour l'insertion à travers le système vasculaire d'un patient, et une configuration dilatée, dans laquelle les première et deuxième parties d'ancrage (5, 6) s'étendent radialement vers l'extérieur à partir de ladite partie centrale de la structure de support (3) pour comprimer une cloison (7) entre elles, en disposant ladite structure de support (2) à cheval sur ladite cloison (7) à travers un défaut, un trou ou une ouverture présent dans la cloison (7) ;
et dans lequel
ledit dispositif d'occlusion interseptal traversable (1) comprend en outre un diaphragme (8) en matériau élastique capable d'une déformation d'au moins 150 % ;
ledit diaphragme (8) étant supporté par ladite structure de support (2) et conçu pour occlure une majorité dudit lumen (4) lorsque ledit diaphragme (8) est dans une configuration relâchée ;
ledit diaphragme (8) comprenant une pluralité de fentes (27) ; et
ledit diaphragme (8) étant configuré pour permettre à un instrument médical inséré dans un premier compartiment (9 ou 10) délimité par ladite cloison (7) de passer à travers le diaphragme (8) par l'une des fentes (27) de ladite pluralité de fentes (27) et ensuite à travers ledit lumen (4) en pénétrant dans un second compartiment (10 ou 9) délimité par ladite cloison (7) ; dans lequel
ledit diaphragme (8) comprend une pluralité de ponts d'occlusion (21) ;
chacun de ladite pluralité de ponts d'occlusion (21) comprend un corps allongé (50)
ayant des extrémités de corps allongées opposées (51, 52) et des bords longitudinaux opposés (22) ;
dans la configuration détendue dudit diaphragme (8), chaque pont d'occlusion (21) de ladite pluralité de ponts d'occlusion (21) est disposé de manière adjacente à au moins un autre pont d'occlusion (21) de ladite pluralité de ponts d'occlusion (21) ;
au moins une partie desdits bords longitudinaux (22) de tout pont d'occlusion (21) et au moins une partie dudit bord longitudinal (22) dudit autre pont d'occlusion (21) délimitant au moins une fente de la pluralité de fentes (27) ;
les deux extrémités opposées (51, 52) de chaque pont d'occlusion (21) sont
directement ou indirectement
reliées à ou supportées par ladite structure de support (2).

2. Dispositif occlusif interseptal traversable (1) selon la revendication 1, dans lequel, dans la configuration détendue dudit diaphragme (8), chaque fente de ladite pluralité de fentes (27) présente une extension longitudinale égale à une extension longitudinale entière dudit corps allongé (50) de chaque pont occlusif (21) délimitant ladite fente (27).

3. Dispositif occlusif interseptal traversable (1) selon l'une quelconque des revendications précédentes, dans lequel lesdits bords longitudinaux (22) des ponts d'occlusion adjacents (21) sont côte à côte et en contact les uns avec les autres.

4. Dispositif occlusif interseptal traversable (1) selon l'une quelconque des revendications précédentes, dans lequel, dans la configuration détendue dudit diaphragme (8), chacune de ladite pluralité de fentes (27) repose dans un plan disposé orthogonalement à un axe dudit lumen (4).

5. Dispositif occlusif interseptal traversable (1) selon l'une quelconque des revendications précédentes, dans lequel les deux extrémités opposées du corps allongé (51, 52) de chaque pont occlusif (21) sont directement ou indirectement reliées à ou supportées par la partie centrale de la structure de support (3) de ladite structure de support (2).

6. Dispositif occlusif interseptal traversable (1) selon l'une quelconque des revendications précédentes, dans lequel ledit diaphragme (8) est une membrane élastique.

7. Dispositif d'occlusion interseptale traversable (1) selon l'une quelconque des revendications précédentes, dans lequel ledit diaphragme (8) est configuré pour empêcher un écoulement de sang à travers ledit lumen (4) dans la configuration détendue.

8. Dispositif occlusif interseptal traversable (1) selon l'une quelconque des revendications précédentes, comprenant en outre un deuxième diaphragme (8), ledit deuxième diaphragme (8) comprenant une deuxième pluralité de fentes (27), dans lequel ledit diaphragme (8) et ledit deuxième diaphragme (8) sont disposés face à face et avec la pluralité de fentes (27) du diaphragme (8) décalées par rapport à la pluralité de fentes (27) du deuxième diaphragme (8).

9. Dispositif d'occlusion interseptale traversable (1) selon l'une quelconque des revendications précédentes, dans lequel ledit diaphragme (8) comprend une pluralité de fils élastiques (42) qui forment ledit diaphragme (8).

10. Dispositif d'occlusion interseptale traversable (1) selon l'une quelconque des revendications 1 à 7 ou la revendication 9, lorsqu'elle ne dépend pas de la revendication 8, comprenant en outre un deuxième diaphragme (8), ledit deuxième diaphragme (8) comprenant une deuxième pluralité de fentes (27), dans lequel ledit diaphragme (8) et ledit deuxième diaphragme (8) sont disposés face à face et avec la pluralité de fentes (27) du diaphragme (8) sensiblement orthogonale par rapport à la pluralité de fentes (27) du deuxième diaphragme (8).

11. Dispositif d'occlusion interseptale traversable (1) selon l'une quelconque des revendications précédentes, dans lequel, dans la configuration détendue dudit diaphragme (8), ladite pluralité de ponts d'occlusion (21) couvre 70-100 % de la lumière (4).

12. Dispositif d'occlusion interseptale traversable (1) selon l'une quelconque des revendications précédentes, dans lequel, dans la configuration détendue, chaque fente de ladite pluralité de fentes (27) présente un diamètre compris entre zéro millimètre et un millimètre.

13. Le dispositif d'occlusion interseptal traversable (1) selon la revendication 12, dans lequel ledit Le diaphragme (8) est configuré pour permettre la repousse naturelle des tissus afin d'occlure la pluralité de fentes (27).

14. Le dispositif d'occlusion interseptale traversable (1) selon l'une quelconque des revendications précédentes, dans lequel, à l'exception des extrémités opposées (51, 52), chaque corps allongé (50) de chaque pont d'occlusion (21) est indépendant des autres corps allongés (50) des autres ponts d'occlusion (21).

15. Le dispositif d'occlusion interseptal traversable (1) selon l'une quelconque des revendications précédentes, dans lequel ledit diaphragme (8) est constitué de silicone, d'un élastomère médical ou de polyuréthane.
